# EUROPÄISCHE PATENTSCHRIFT

(11) **EP 2 209 760 B1**
(45) Veröffentlichungstag und Bekanntmachung des Hinweises auf die Patenterteilung: **13.03.2013**
(21) Anmeldenummer: 08803358.4
(22) Anmeldetag: 29.08.2008
(51) Int. Cl.: B01D 1/00, B01D 1/22, C07C 67/54, C07C 69/54

(54) **VERFAHREN UND ANLAGE ZUR AUFREINIGUNG VON UNGESÄTTIGTEN VERBINDUNGEN**
METHOD AND PLANT FOR PURIFYING UNSATURATED COMPOUNDS
PROCEDE ET INSTALLATION DE PURIFICATION DE COMPOSES INSATURES

(30) Priorität: 23.11.2007 DE 102007056926
(43) Veröffentlichungstag der Anmeldung: 28.07.2010
(73) Patentinhaber: Evonik Röhm GmbH, 64293 Darmstadt (DE)
(72) Erfinder: LAUX, Benedikt, 55234 Monzernheim (DE); MAUL, Christian, 67435 Neustadt a. d. W. (DE); SCHLEEP, Volker, 64683 Einhausen (DE); SANDER, Ingo, 201108 Shangai (CN)
(86) Internationale Anmeldenummer: PCT/EP2008/061359
(87) Internationale Veröffentlichungsnummer: WO 2009/065633

(56) Entgegenhaltungen:
- EP-A- 1 090 904
- WO-A-00/08072
- WO-A-2004/063140

## Beschreibung

Die vorliegende Erfindung betrifft ein Verfahren zur Aufreinigung von ungesättigten Verbindungen. Darüber hinaus beschreibt die vorliegende Erfindung eine Anlage zur Durchführung des vorliegenden Verfahrens.

Ungesättigte Verbindungen mit hoher Siedetemperatur neigen bei der Aufreinigung zur Bildung von Nebenprodukten. Hierdurch ergeben sich vielfältige Probleme im Zusammenhang mit der Isolierung dieser Verbindungen. Beispielsweise können sich durch Polymerisation der ungesättigten Verbindungen Ablagerungen in den Aufreinigungsanlagen bilden. Um eine hohe Qualität des Produkts sicherzustellen, ist es daher notwendig diese Anlagen von Zeit zu Zeit zu reinigen.

### Stand der Technik

Darüber hinaus führt die Bildung von Nebenprodukten zu einer Verringerung der Gesamtausbeute. Bei einer Aufreinigung in einer Destille mit einer Kolonne, die aufgrund weniger Einbauten einen geringen Druckabfall aufweist, können diese Probleme vermieden werden. Hierdurch kann jedoch keine optimale Trennleistung erzielt werden. Andererseits ist es vielfach notwendig ein möglichst reines Produkt zur Verfügung zu stellen. Zu den besonders problematischen Verbindungen gehören insbesondere Glykolester, wie beispielsweise Hydroxyalkyl(meth)acrylate. Neben Polymeren können diese Verbindungen Di(meth)acrylate bilden, die besonders unerwünscht sind. Um die zuvor dargelegten Probleme zu lösen, wurden bereits Anstrengungen unternommen.

WO 2004/063140 beschreibtein Verfahen zur Aufreiningung ungesöttigten Verbindungen.

Beispielsweise beschreibt die Druckschrift CN1502601 ein Verfahren zur Aufreinigung von Hydroxyalkyl(meth)acrylaten, das mehrere Stufen aufweist. Die einzelnen Stufen sind hierbei in Serie geschaltet. In einem ersten Dünnschichtverdampfer werden die schwer flüchtigen Verbindungen abgetrennt. Das verdampfte Produkt wird kondensiert und in einen Fallfilmverdampfer geleitet. Im Fallfilmverdampfer wird die zu reinigende Verbindung von leicht flüchtigen Bestandteilen gereinigt. In einem weiteren Dünnschichtverdampfer wird das Produkt über Kopf abgezogen, schwerflüchtige Bestandteile werden mit einem geringen Produktanteil zum ersten Dünnschichtverdampfer zurückgeführt. Das Produkt wird dementsprechend nicht in einem Kreislauf geführt. Ein Hydroxypropylmethacrylat, das gemäß dem in der Druckschrift CN1502601 beschriebenen Verfahren gereinigt wurde, zeigte eine Reinheit von ca. 98,7 %.

Des Weiteren beschreibt das Dokument EP-A-1 090 904 ein Verfahren zur Aufreinigung von Hydroxyalkyl(meth)acrylaten. In diesem Verfahren wird eine Destille zusammen mit einem Dünnschichtverdampfer eingesetzt, wobei der Sumpf der Destille in den Dünnschichtverdampfer eingeleitet wird. Die Kolonne der Destille weist keine Einbauten auf, die zu einem Druckabfall führen. Durch diese Maßnahmen kann erreicht werden, dass das aufgereinigte Produkt eine relativ hohe Reinheit bei einem geringen Diesteranteil aufweist. Im Vergleich zu einer reinen Destillation nimmt die Reinheit jedoch nur unwesentlich von 98,1 auf 98,5% zu. Wesentlich ist hierbei, dass die Verweilzeit klein gehalten werden kann. Die Rückführung einer großen Menge an verdampftem Produkt aus dem Dünnschichtverdampfer in die Destille wird nicht quantitativ beschrieben.

Die zuvor dargelegten Verfahren zeigen bereits eine Verbesserung gegenüber dem herkömmlichen Stand der Technik. Allerdings besteht das dauerhafte Bedürfnis die Reinheit sowie die Ausbeute der ungesättigten Verbindungen weiter zu verbessern.

### Aufgabe

In Anbetracht des Standes der Technik ist es nun eine Aufgabe der vorliegenden Erfindung, Verfahren zur Aufreinigung von ungesättigten Verbindungen zur Verfügung zu stellen, die bei einer hohen Ausbeute zu besonders reinen Produkten führen.

Eine weitere Aufgabe bestand insbesondere darin ein Verfahren zu schaffen, bei dem die Bildung von Nebenprodukten bei der Aufreinigung besonders gering ist. Insbesondere sollte das Verfahren in den Anlagen zur Aufreinigung nur zu einer geringen Bildung von Ablagerungen führen. Hierdurch sollte ein langer Dauerbetrieb der Anlage ermöglicht werden, ohne dass der Betrieb der Anlage wegen Reinigungsmaßnahmen unterbrochen werden muss.

Weiterhin sollte das Verfahren möglichst einfach und kostengünstig durchgeführt werden können.

Darüber hinaus war es mithin eine Aufgabe der vorliegenden Erfindung, eine Anlage zur Durchführung eines entsprechenden Verfahrens zur Verfügung zu stellen. Hierbei sollte die Anlage auf einfache Weise gereinigt werden können.

### Lösung

Gelöst werden diese sowie weitere nicht explizit genannte Aufgaben, die jedoch aus den hierin einleitend diskutierten Zusammenhängen ohne weiteres ableitbar oder erschließbar sind, durch ein Verfahren mit allen Merkmalen des Patentanspruchs 1. Zweckmäßige Abwandlungen des erfindungsgemäßen Verfahrens werden in Unteransprüchen unter Schutz gestellt. Bezüglich der Anlage zur Durchführung des Verfahrens stellt der Anspruch 20 eine Lösung des Problems dar.

Gegenstand der vorliegenden Erfindung ist dementsprechend ein Verfahren zur Aufreinigung von ungesättigten Verbindungen, wobei die Aufreinigung in einer Anlage durchgeführt wird, die mindestens zwei Verdampfer aufweist und die Verdampfer so verbunden sind, dass ein Teil der ungesättigten Verbindung in einem Kreislauf geführt wird, wobei die im ersten Verdampfer kondensierten Brüden isoliert werden und die im zweiten Verdampfer kondensierten Brüden in den ersten Verdampfer eingeleitet werden, welches dadurch gekennzeichnet ist, dass der Massenstrom, mit dem die kondensierten Brüden in dem ersten Verdampfer aus der zu reinigenden Mischung isoliert werden, kleiner ist als der Massenstrom, mit dem die kondensierten Brüden aus dem zweiten Verdampfer in den ersten Verdampfer eingeleitet werden.

Hierdurch gelingt es, auf nicht vorhersehbare Weise ein Verfahren der zuvor dargelegten Gattung bereitzustellen, dass ein besonders gutes Eigenschaftsprofil aufweist. Überraschend können insbesondere Produkte mit einer besonders hohen Reinheit bei einer sehr guten Ausbeute erhalten werden.

Weiterhin ist die Bildung von Nebenprodukten bei der Aufreinigung besonders gering. Hierbei führt das Verfahren in den Anlagen zur Aufreinigung nur zu einer geringen Bildung von Ablagerungen. Hierdurch gelingt ein langer Dauerbetrieb der Anlagen, ohne dass der Betrieb der Anlage unterbrochen werden muss.

Darüber hinaus kann das erfindungsgemäße Verfahren sehr einfach und kostengünstig durchgeführt werden.

Ferner stellt die vorliegende Erfindung eine Anlage zur Durchführung eines entsprechenden Verfahrens zur Verfügung, wobei die Anlage auf einfache Weise gereinigt werden kann.

Das Verfahren der vorliegenden Erfindung dient insbesondere zur Aufreinigung von ungesättigten Verbindungen, die mindestens eine Kohlenstoff-Kohlenstoff-Doppelbindung aufweisen. Von besonderem Interesse sind insbesondere Verbindungen, die insbesondere einen Siedepunkt bei 1.013 mbar von ca. 150 °C, bis ca. 300°C aufweisen. Zweckmäßig kann das erfindungsgemäße Verfahren insbesondere zur Aufreinigung von Glykolestern eingesetzt werden. Hierzu gehören beispielsweise Hydroxyalkyl(meth)acrylate, wie 2-Hydroxyethyl(meth)acrylat, Hydroxypropyl(meth)acrylat und Isomere, Hydroxybutyl(meth)acrylat und Isomere sowie Mischungen aus den vorgenannten Verbindungen. Die Herstellung dieser Verbindungen ist allgemein bekannt, wobei sich wertvolle Hinweise insbesondere im zuvor zitierten Stand der Technik finden. So können diese Verbindungen insbesondere durch die Umsetzung von (Meth)acrylsäure mit Epoxiden, beispielsweise Ethylenoxid oder Propylenoxid erhalten werden.

Die Aufreinigung kann in Gegenwart von Stabilisatoren erfolgen, wobei diese vielfach bereits zur Herstellung der ungesättigten Verbindungen eingesetzt werden können. Viele aufzutrennende Mischungen weisen daher bereits diese Verbindungen auf, wobei diese gegebenenfalls hinzugefügt werden können. Zu den bevorzugten Stabilisatoren gehören unter anderem Phenolverbindungen, wie zum Beispiel Hydrochinon, Methylhydrochinon, tert.-Butylhydrochinon, 2,6-Di-tert-butylhydrochinon, 2,5-Di-tert-butylhydrochinon, 2,4-Dimethyl-6-tert-butylphenol und Hydrochinonmonomethylether; p-Phenylendiamine, wie zum Beispiel N-Isopropyl-N'-phenyl-p-phenylendiamin, N-(1,3-Dimethylbutyl)-N'-phenyl-p-phenylendiamin, N-(1-Methylheptyl)-N'-phenyl-p-phenylendiamin, N,N'-Diphenyl-p-phenylendiamin und N,N'-Di-2-naphthyl-p-phenylendiamin; Amine, wie zum Beispiel Thiodiphenylamin und Phenothiazine; Kupfer-Dialkyldithiocarbamate, wie zum Beispiel Kupferdibutyldithiocarbamat, Kupferdiethyldithiocarbamat und Kupferdimethyldithiocarbamat; Nitrosoverbindungen, wie zum Beispiel Nitrosodiphenylamin, Isoamylnitrit, N-Nitrosocyclohexylhydroxylamin, N-Nitroso-N-phenyl-N-hydroxylamin und deren Salze; und N-Oxylverbindungen, wie zum Beispiel 2,2,4,4-Tetramethylazetidin-1-oxyl, 2,2-Dimethyl-4,4-dipropylazetidin-1-oxyl, 2,2,5,5-Tetramethylpyrrolidin-1-oxyl, 2,2,5,5-Tetramethyl-3-oxopyrrolidin-1-oxyl, 2,2,6,6-Tetramethylpiperidin-1-oxyl, 4-Hydroxy-2,2,6,6-tetramethylpiperidin-1-oxyl, 6-Aza-7,7-dimethylspiro[4,5]decan-6-oxyl, 2,2,6,6-Tetramethyl-4-acetoxypiperidin-1-oxyl und 2,2,6,6-Tetramethyl-4-benzoyloxypiperidin-1-oxyl. Diese Verbindungen können bevorzugt in einer Menge im Bereich von 0,0001 Gew.-% bis 1 Gew.-%, besonders bevorzugt im Bereich von 0,0005 Gew.-% bis 0,5 Gew.-% in der zu reinigenden Mischung enthalten sein.

Die Aufreinigung wird in einer Anlage durchgeführt, die mindestens zwei Verdampfer aufweist. Der Ausdruck "Verdampfer" bezeichnet im Rahmen der vorliegenden Erfindung eine Vorrichtung, die zum Überführen der ungesättigten Verbindung in die Gasphase geeignet ist. Hierzu gehören insbesondere Dünnschichtverdampfer, wie beispielsweise Fallfilmverdampfer und Verdampfer mit einem rotierenden Wischersystem, sowie Umlaufverdampfer, wie beispielsweise Zwangs- oder Naturumlaufverdampfer. Auch Kurzwegverdampfer können eingesetzt werden. Solche Vorrichtungen sind bekannt (vgl. Ullmanns Encyclopedia of Industrial Chemistry (6. Auflage), Verlag Wiley-VCH, Weinheim 2003, Band 36, Seite 505).

In der Anlage zur Durchführung des erfindungsgemäßen Verfahrens sind mindestens zwei Verdampfer so verbunden, dass ein Teil der ungesättigten Verbindung in einem Kreislauf geführt werden kann, wobei die im ersten Verdampfer kondensierten Brüden isoliert werden und die im zweiten Verdampfer kondensierten Brüden in den ersten Verdampfer eingeleitet werden. Wie bereits dargelegt sind die Verdampfer so miteinander verbunden, dass ein Teil der ungesättigten Verbindungen in einem Kreislauf geführt werden kann. Dies bedeutet, dass der in dem ersten Verdampfer verbliebene Teil der Zusammensetzung, der nicht in die Gasphase überführt wurde, in den zweiten Verdampfer überführt wird, beispielsweise in den Sumpf. Dieser Sumpf umfasst insbesondere einen hohen Anteil an schwerflüchtigen Verbindungen. Erfindungsgemäß ist der Massenstrom, mit dem die kondensierten Brüden in dem ersten Verdampfer aus der zu reinigenden Mischung isoliert werden, kleiner als der Massenstrom, mit dem die kondensierten Brüden aus dem zweiten Verdampfer in den ersten Verdampfer eingeleitet werden.
Von besonderem Interesse sind insbesondere Verfahren, bei denen das Verhältnis des Massenstromes, mit dem die kondensierten Brüden in dem ersten Verdampfer aus der zu reinigenden Mischung isoliert werden (3), zu dem Massenstrom, mit dem die kondensierten Brüden aus dem zweiten Verdampfer in den ersten Verdampfer eingeleitet werden (6), vorzugsweise im Bereich von 0,4 bis 1,5, besonders bevorzugt im Bereich von 0,5 bis 0,8 liegt. Hieraus ergibt sich, dass ein hoher Anteil der in den ersten Verdampfer eingeleiteten Zusammensetzungen (2) über den Sumpf in den zweiten Verdampfer eingeleitet wird (4). Dieser Anteil ergibt sich aus den zuvor dargelegten Verhältnissen der zwei Massenströme.

Gemäß einer besonderen Ausführungsform kann das erfindungsgemäße Verfahren in einer Anlage durchgeführt werden, die mindestens drei Verdampfer aufweist, wobei ein Kreislauf zwischen dem ersten und dem zweiten Verdampfer (4 & 6) und ein Kreislauf zwischen dem zweiten und dem dritten Verdampfer (7 & 9) gebildet werden, wobei die zuvor dargelegten Ausführungen bezüglich der Kreisläufe entsprechend gelten.
Hierbei kann das Verhältnis des Massenstromes, mit dem die kondensierten Brüden aus dem zweiten Verdampfer in den ersten Verdampfer eingeleitet werden (6), zu dem Massenstrom, mit dem die kondensierten Brüden aus dem dritten Verdampfer in den zweiten Verdampfer eingeleitet werden (9), vorzugsweise im Bereich von 0,75 bis 35, besonders bevorzugt im Bereich von 2 bis 10 liegen. Durch diese Ausgestaltung des erfindungsgemäßen Verfahrens kann insbesondere die Ausbeute überraschend gesteigert werden.

Die zu reinigende Mischung kann vorzugsweise in den ersten und/oder den zweiten Verdampfer eingeleitet werden. Von besonderem Interesse sind insbesondere Verfahren, bei denen mindestens ein Teil der zu reinigenden Mischung zunächst in den ersten Verdampfer und ein weiterer Teil der zu reinigenden Mischung zunächst in den zweiten Verdampfer eingeleitet werden. Zweckmäßig kann das Massenstromverhältnis des in den ersten Verdampfer eingeleiteten Teils der zu reinigenden Mischung zu dem in den zweiten Verdampfer eingeleiteten Teil der zu reinigenden Mischung vorzugsweise im Bereich von 9:1 bis 0,1:1, besonders bevorzugt im Bereich von 8:1 bis 2:1 liegen.

Die Art der Verdampfer ist für die Durchführung des erfindungsgemäßen Verfahrens an sich von untergeordneter Bedeutung. Es hat sich allerdings als zweckmäßig erwiesen, dass ein Umlaufverdampfer als erster und zweiter Verdampfer eingesetzt wird. Als dritter Verdampfer wird vorzugsweise ein Dünnschichtverdampfer verwendet.

Das erfindungsgemäße Aufreinigungsverfahren kann in einem weiten Druck-und Temperaturbereich ausgeführt werden. Um die Bildung von Nebenprodukten möglichst gering zu halten, ist es zweckmäßig die Temperatur auf einem geringen Niveau zu halten. Dementsprechend wird das erfindungsgemäße Verfahren vorzugsweise bei einem geringen Druck ausgeführt. Andererseits gestaltet sich das Aufrechterhalten eines sehr geringen Drucks als sehr aufwendig.

Besonders zweckmäßig wird daher die ungesättigte Verbindung vorzugsweise bei einem Druck im Bereich von 0,1 mbar bis 20 mbar, besonders bevorzugt im Bereich von 1 mbar bis 10 mbar in die Gasphase überführt.

Hierbei notwendigen Temperaturen ergeben sich aus der Dampf-Druckkurve der jeweiligen ungesättigten Verbindung. Im Allgemeinen liegen diese Temperaturen, bei der die ungesättigten Verbindungen in die Gasphase überführt werden, vorzugsweise im Bereich von 50 °C bis 150 °C, besonders bevorzugt 60 °C bis 110 °C.

Gemäß einem besonderen Aspekt der vorliegenden Erfindung wird der erste Verdampfer vorzugsweise bei einem Gasbelastungsfaktor von 1,5 bis 2 Pa^{0,5} betrieben. Zweckmäßig liegt der Gasbelastungsfaktor, bei dem der erste Verdampfer betrieben wird bevorzugt im Bereich von 0,8 bis 3,0 Pa^{0,5}. Der Gasbelastungsfaktor (F-Faktor) berechnet sich aus der auf den leeren Querschnitt des Rohres zur Ableitung des Gases bezogenen Gasgeschwindigkeit multipliziert mit der Wurzel der Gasdichte (vgl. Klaus Sattler, Till Adrian, Thermische Trennverfahren (3. Auflage), VCH-Verlag, Weinheim 2001, Seite 234).

Die vorliegende Erfindung stellt des Weiteren eine bevorzugte Anlage zur Durchführung des erfindungsgemäßen Verfahrens zur Verfügung. Eine erfindungsgemäße Anlage weist mindestens drei Verdampfer auf, die so miteinander verbunden sind, dass der in der flüssigen Phase verbliebene Rückstand des ersten Verdampfers in den zweiten Verdampfer geleitet werden kann, die kondensierten Brüden des zweiten Verdampfers in den ersten Verdampfer und der in der flüssigen Phase verbliebene Rückstand des zweiten Verdampfers in den dritten Verdampfer geleitet werden können, und die kondensierten Brüden des dritten Verdampfers in den zweiten Verdampfer geleitet werden können.

Gemäß einer besonderen Ausführungsform der vorliegenden Anlage weist diese mindestens zwei Umlaufverdampfer und einen Dünnschichtverdampfer auf. Zweckmäßig wird die zu reinigende Mischung zunächst in die Umlaufverdampfer geleitet, wobei vorzugsweise ein Dünnschichtverdampfer als dritter Verdampfer eingesetzt wird.

Die in den Verdampfern erzeugten Brüden können durch übliche Verbindungen in die weiteren Bestandteile der Anlage geleitet werden. Im Allgemeinen können insbesondere Rohre verwendet werden, um das Gas nach dem Verdampfen abzuleiten. Vorzugsweise weist jeder Verdampfer eine Kolonne auf, so dass die Anlage mindestens drei Kolonnen umfasst. Der Begriff Kolonne ist hierbei umfassend zu verstehen, so dass hierunter auch einfache Rohre umfasst werden. Gemäß einem besonderen Aspekt der vorliegenden Erfindung können insbesondere Kolonnen mit einem Durchmesser von 0,05 m bis 5 m, besonders bevorzugt im Bereich von 0,3 m bis 3 m eingesetzt werden. Zweckmäßig werden hierbei insbesondere Kolonnen eingesetzt, die einen geringen Druckabfall bewirken. Von besonderem Interesse sind insbesondere Kolonnen, die bei einem F-Faktor im Bereich von 0,8 bis 3,0 Pa^{0,5} vorzugsweise im Bereich von 1,5 bis 2,0 Pa^{0,5} zu einem Druckabfall im Bereich von 0,5 bis 10 vorzugsweise im Bereich von 1 mbar bis 5 mbar führen. Dementsprechend werden vorzugsweise Kolonnen eingesetzt, die keine Packungen oder Einbauten aufweisen. Ausgenommen sind jedoch Tröpfchenabscheider (Demister), die dazu dienen Verbindungen, die bei den gewählten Bedingungen im Allgemeinen nicht verdampfen, aber in Tröpfchen mitgerissen werden können, im Sumpf zurückzuhalten.

Vorzugsweise können die in die Gasphase überführten Zusammensetzungen kondensiert werden (kondensierte Brüden), bevor diese in den nächsten Verdampfer eingeleitet werden. Dementsprechend können zwischen den verschiedenen Verdampfern Kondensatoren angeordnet sein. Dementsprechend kann eine bevorzugte Anlage zur Durchführung der vorliegenden Erfindung drei miteinander verbundene Destillen umfassen.

Die vorliegende Erfindung ist jedoch nicht auf eine Anlage mit drei Verdampfern bzw. drei Destillen begrenzt. Gemäß weiteren Ausführungsformen kann eine erfindungsgemäße Anlage vier, fünf oder mehr Verdampfer aufweisen, die entsprechend dem zuvor dargelegten Prinzip miteinander verbunden sind, so dass zwischen zwei Verdampfern jeweils ein Kreislauf gebildet wird.

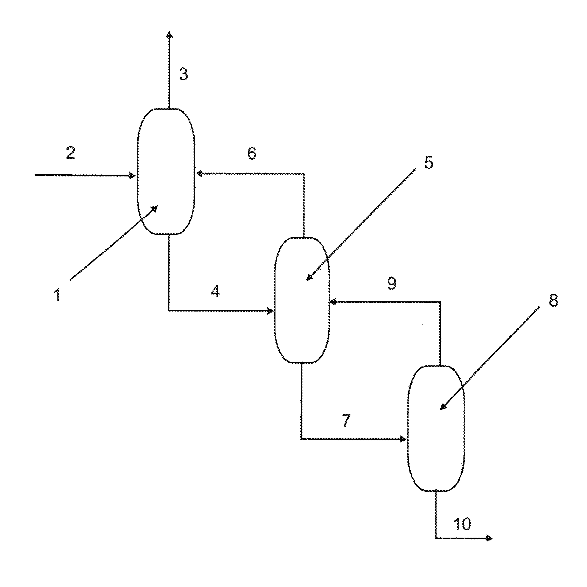

Zur näheren Erläuterung wird die vorliegende Erfindung anhand der Figur 1 beschrieben, ohne dass hierdurch eine Beschränkung der Erfindung erfolgen soll.

Figur 1 zeigt in einer schematischen Darstellung eine bevorzugte Anlage zur Durchführung der vorliegenden Erfindung. In einen ersten Umlaufverdampfer (1) wird über Massenstrom (2) das aus einer Produktion erhaltene Hydroxyalkyl(meth)acrylat eingeleitet. Der Umlaufverdampfer kann bei einem Druck im Bereich von 1 mbar bis 10 mbar betrieben werden, wobei sich je nach Ester ein Temperaturbereich von 55°C bis 120°C ergibt. Das Destillat wird kondensiert und über Massenstrom (3) aus der Anlage geleitet. Der hierfür notwendige Kondensator kann mit dem Verdampfer eine Einheit bilden oder als separate Einheit ausgestaltet sein. Der nicht verdampfte Anteil des zugeleiteten Esters wird über Massenstrom (4) in den zweiten Umlaufverdampfer (5) geleitet, in dem ein Großteil des zugeleiteten Esters verdampft wird und nach Kondensation über Massenstrom (6) in den ersten Umlaufverdampfer (1) überführt wird. Der im zweiten Umlaufverdampfer (5) gebildete Rückstand wird über Massenstrom (7) in den Dünnschichtverdampfer (8) geleitet. In Dünnschichtverdampfer (8) wird ebenfalls ein Teil der zugeleiteten Mischung in die Gasphase überführt, kondensiert und über Massenstrom (9) in den zweiten Umlaufverdampfer (5) eingeleitet. Der in Dünnschichtverdampfer (8) verbliebene Rest wird über Massenstrom (10) aus der Anlage geschleust.

Die Verdampfer (5) beziehungsweise (8) werden vorzugsweise ebenfalls bei einem Druck im Bereich von 1 bis 10 mbar und einer Temperatur im Bereich von 55 bis 120°C betrieben. Vorzugsweise wird die Anlage so eingestellt, dass das Verhältnis des Massenstromes, mit dem die gereinigte Verbindung über Massenstrom (3) aus der Anlage ausgeschleust wird, zum Massenstrom, mit dem das aus einer Produktion erhaltene Hydroxyalkyl(meth)acrylat über Massenstrom (2) in die Anlage eingeleitet wird, im Bereich von 0,85 bis 0,97, bevorzugt 0,90 bis 0,95 liegt.

Mit besonderem Vorteil werden hoch siedende Verbindungen mit einem hohen Massenstrom aus dem ersten Umlaufverdampfer (1) durch Leitung (4) in den zweiten Umlaufverdampfer (5) überführt. Das Verhältnis des Massenstromes mit dem hoch siedende Verbindungen aus dem ersten Umlaufverdampfer (1) durch Leitung (4) in den zweiten Umlaufverdampfer (5) überführt werden, zum Massenstrom mit dem das aus einer Produktion erhaltene Hydroxyalkyl(meth)acrylat über Leitung (2) in die Anlage eingeleitet wird, liegt vorzugsweise im Bereich von 0,75 bis 2, besonders bevorzugt im Bereich von 1,20 bis 1,80.

Zweckmäßig liegt das Verhältnis des Massenstromes, mit dem die in den zweiten Umlaufverdampfer (5) kondensierten Brüden über Leitung (6) in den ersten Umlaufverdampfer (1) überführt werden, zum Massenstrom mit dem das aus einer Produktion erhaltene Hydroxyalkyl(meth)acrylat über Leitung (2) in die Anlage eingeleitet wird, im Bereich von 0,75 bis 1,90, besonders bevorzugt im Bereich von 1,25 bis 1,75, ohne dass hierdurch eine Beschränkung erfolgen soll.

Der im zweiten Umlaufverdampfer (5) erhaltene Rückstand hoch siedender Verbindungen wird in den Dünnschichtverdampfer (8) eingeleitet. Hierbei wird die Anlage vorzugsweise so eingestellt, dass das Verhältnis des Massenstromes, mit dem diese hoch siedenden Verbindungen über Leitung (7) in den Dünnschichtverdampfer (8) überführt werden, zum Massenstrom, mit dem das aus einer Produktion erhaltene Hydroxyalkyl(meth)acrylat über Leitung (2) in die Anlage eingeleitet wird, im Bereich von 0,05 bis 1,25, besonders bevorzugt 0,10 bis 0,50 liegt.

Die in den Dünnschichtverdampfer (8) gebildete Gasphase wird nach Kondensation in den zweiten Umlaufverdampfer (5) überführt. Vorzugsweise liegt das Verhältnis des Massenstromes, mit dem die kondensierten Brüden in den zweiten Umlaufverdampfer (5) überführt werden, zum Massenstrom, mit dem das aus einer Produktion erhaltene Hydroxyalkyl(meth)acrylat über Leitung (2) in die Anlage eingeleitet wird, im Bereich von 0,01 bis 1,20, besonders bevorzugt 0,05 bis 0,50.

Der in Dünnschichtverdampfer (8) erhaltene Rückstand wird aus der Anlage ausgeschleust. Zweckmäßig beträgt das Verhältnis des Massenstromes, mit dem der in Dünnschichtverdampfer (8) erhaltene Rückstand aus der Anlage ausgeschleust wird, zum Massenstrom, mit dem das aus einer Produktion erhaltene Hydroxyalkyl(meth)acrylat über Leitung (2) in die Anlage eingeleitet wird, 0,03 bis 0,15, besonders bevorzugt 0,05 bis 0,10.

Die Anlage gemäß Figur 1 wurde im Zusammenhang mit Hydroxyalkyl(meth)acrylaten beschrieben. Die Massenströme, insbesondere die Verhältnisse der einzelnen Massenströme gelten in gleicher Weise für andere ungesättigte Verbindungen, insbesondere andere Glykolester. Gleiches gilt für Anlagen, die andere Verdampfertypen oder mehr als drei Verdampfer umfassen. Falls die zu reinigende Mischung über mehr als einen Massenstrom in die Anlage geleitet wird, beziehen sich die zuvor dargelegten Werte auf die Massenströme mit der die Gesamtmenge in die Anlage geleitet wird.

Nachfolgend wird die Erfindung anhand von Beispielen näher erläutert, ohne dass hierdurch eine Beschränkung erfolgen soll.

### Beispiel 1

In einer in Figur 1 dargelegten Anlage wurde 2-Hydroxyethylmethacrylat in einem Dauerversuch, der über 14 Tage durchgeführt wurde, aufgereinigt. Der erste Umlaufverdampfer (1) wurde mit einem Druck von circa 2 mbar - 3 mbar und einer Temperatur von circa 66°C - 72°C betrieben. Die Zulaufmenge, mit der das aus einer zuvor durchgeführten Produktion erhaltene 2-Hydroxyethylmethacrylat über Leitung (2) in die Anlage eingeleitet wurde, betrug im Mittel 1,605 kg/h. Der Massenstrom, mit dem die erhaltenen Brüden aus dem ersten Umlaufverdampfer (1) aus der Anlage über Leitung (3) ausgeleitet wurden, betrug im Mittel 1,518 kg/h.

Der im ersten Umlaufverdampfer (1) erhaltene Rückstand wurde mit einem Massenstrom von im Mittel 1,930 kg/h über Leitung (4) in den zweiten Umlaufverdampfer (5) geleitet. Der zweite Umlaufverdampfer (5) wurde bei einem Druck von circa 2mbar - 3 mbar und einer Temperatur von circa 66°C - 74 °C betrieben, wobei die kondensierten Brüden mit einem Massenstrom von im Mittel 1,843 kg/h über Leitung (6) in den ersten Umlaufverdampfer (1) geleitet wurden.

Der im zweiten Umlaufverdampfer (5) erhaltene Rückstand wurde mit einem Massenstrom von im Mittel 0,580 kg/h über Leitung (7) in den Dünnschichtverdampfer (8) geleitet. Der Dünnschichtverdampfer (8) wurde bei einem Druck von circa 2 mbar - 3 mbar und einer Temperatur von circa 68°C - 78 °C betrieben, wobei die kondensierten Brüden mit einem Massenstrom von im Mittel 0,493 kg/h über Leitung (9) in den Umlaufverdampfer (5) geleitet wurden. Der erhaltene Rückstand von im Mittel 0,087 kg/h wurde über Leitung (10) aus der Anlage ausgeschleust.

Das in die Anlage über Leitung (2) eingeleitete 2-Hydroxyethylmethacrylat wies eine Reinheit von circa 95,0 %. Das aufgereinigte 2-Hydroxyethylmethacrylat zeigte eine Reinheit von 99,1 %. Die Ausbeute betrug 94,6 %.
Die Anlage zeigte nach diesem Dauerversuch keine Spuren von gebildetem Polymer.

### Beispiel 2

In einer in Figur 1 dargelegten Anlage wurde Hydroxypropylmethacrylat in einem Dauerversuch, der über 15 Tage durchgeführt wurde, aufgereinigt. Der erste Umlaufverdampfer (1) wurde mit einem Druck von circa 2 mbar - 3 mbar und einer Temperatur von circa 68°C - 78°C betrieben. Die Zulaufmenge, mit der das aus einer zuvor durchgeführten Produktion erhaltene Hydroxypropylmethacrylat über Leitung (2) in die Anlage eingeleitet wurde, betrug im Mittel 1,486 kg/h. Der Massenstrom, mit dem die erhaltenen Brüden aus dem ersten Umlaufverdampfer (1) aus der Anlage über Leitung (3) ausgeleitet wurden, betrug im Mittel 1,403 kg/h.

Der im ersten Umlaufverdampfer (1) erhaltene Rückstand wurde mit einem Massenstrom von im Mittel 1,540 kg/h über Leitung (4) in den zweiten Umlaufverdampfer (5) geleitet. Der zweite Umlaufverdampfer (5) wurde bei einem Druck von circa 2 mbar - 3 mbar und einer Temperatur von circa 70°C - 78 °C betrieben, wobei die kondensierten Brüden mit einem Massenstrom von im Mittel 1,475 kg/h über Leitung (6) in den ersten Umlaufverdampfer (1) geleitet wurden.

Der im zweiten Umlaufverdampfer (5) erhaltene Rückstand wurde mit einem Massenstrom von im Mittel 0,680 kg/h über Leitung (7) in den Dünnschichtverdampfer (8) geleitet. Der Dünnschichtverdampfer (8) wurde bei einem Druck von circa 2mbar - 3 mbar und einer Temperatur von circa 72°C - 82 °C betrieben, wobei die kondensierten Brüden mit einem Massenstrom von im Mittel 0,615 kg/h über Leitung (9) in den Umlaufverdampfer (5) geleitet wurden. Der erhaltene Rückstand von im Mittel 0,065 kg/h wurde über Leitung (10) aus der Anlage ausgeschleust.

Das in die Anlage über Leitung (2) eingeleitete Hydroxypropylmethacrylat wies eine Reinheit von circa 95,8 % auf. Das aufgereinigte Hydroxypropylmethacrylat zeigte eine Reinheit von 99,1 % an Zielprodukt. Die Ausbeute betrug 94,4 %. Die Anlage zeigte nach diesem Dauerversuch keine Spuren von gebildetem Polymer.

### Beispiel 3

In einer in Figur 1 dargelegten Anlage wurde 2-Hydroxyethylacrylat in einem Dauerversuch, der über 11 Tage durchgeführt wurde, aufgereinigt. Der erste Umlaufverdampfer (1) wurde mit einem Druck von circa 2 mbar - 3 mbar und einer Temperatur von circa 58°C - 68°C betrieben. Die Zulaufmenge, mit der das aus einer zuvor durchgeführten Produktion erhaltene 2-Hydroxyethylacrylat über Leitung (2) in die Anlage eingeleitet wurde, betrug im Mittel 1,330 kg/h. Der Massenstrom, mit dem die erhaltenen Brüden aus dem ersten Umlaufverdampfer (1) aus der Anlage über Leitung (3) ausgeleitet wurden, betrug im Mittel 1,060 kg/h.

Der im ersten Umlaufverdampfer (1) erhaltene Rückstand wurde mit einem Massenstrom von im Mittel 2,010 kg/h über Leitung (4) in den zweiten Umlaufverdampfer (5) geleitet. Der zweite Umlaufverdampfer (5) wurde bei einem Druck von circa 2 mbar - 3 mbar und einer Temperatur von circa 60°C - 68 °C betrieben, wobei der kondensierten Brüden mit einem Massenstrom von im Mittel 1,740 kg/h über Leitung (6) in den ersten Umlaufverdampfer (1) geleitet wurden.

Der im zweiten Umlaufverdampfer (5) erhaltene Rückstand wurde mit einem Massenstrom von im Mittel 0,930 kg/h über Leitung (7) in den Dünnschichtverdampfer (8) geleitet. Der Dünnschichtverdampfer (8) wurde bei einem Druck von circa 2mbar - 3 mbar und einer Temperatur von circa 62°C - 72 °C betrieben, wobei die kondensierten Brüden mit einem Massenstrom von im Mittel 0,660 kg/h über Leitung (9) in den Umlaufverdampfer (5) geleitet wurde. Der erhaltene Rückstand von im Mittel 0,270 kg/h wurde über Leitung (10) aus der Anlage ausgeschleust.

Das in die Anlage über Leitung (2) eingeleitete 2-Hydroxyethylacrylat wies eine Reinheit von circa 85,3 % auf. Das aufgereinigt 2-Hydroxyethylacrylat zeigte eine Reinheit von 99,0 %. Die Ausbeute betrug 79,7%. Die Anlage zeigte nach diesem Dauerversuch nur ganz geringe Spuren von gebildetem Polymer.

### Vergleichsbeispiel 1

In einer Anlage, bestehend aus einem Umlaufverdampfer und einer Kolonne wurde 2-Hydroxyethylmethacrylat in einem Dauerversuch, der über 14 Tage durchgeführt wurde, aufgereinigt. Die Anlage wurde mit einem Druck von circa 2 mbar - 3 mbar und einer Temperatur von circa 65°C - 73°C betrieben. Die Zulaufmenge, mit der das aus einer zuvor durchgeführten Produktion erhaltene 2-Hydroxyethylmethacrylat in die Mitte der Kolonne eingeleitet wurde, betrug im Mittel 1,605 kg/h. Der Massenstrom, mit dem die erhaltenen Brüden über Kopf aus der Kolonne ausgeleitet wurden, betrug im Mittel 1,495 kg/h. Der erhaltene Rückstand von im Mittel 0,110 kg/h wurde aus dem Umlaufverdampfer aus der Anlage ausgeschleust.

Das in die Anlage eingeleitete 2-Hydroxyethylmethacrylat wies eine Reinheit von circa 95,0 %. Das aufgereinigte 2-Hydroxyethylmethacrylat zeigte eine Reinheit von 98,5 %. Die Ausbeute betrug 93,1 %.
Die Anlage zeigte nach diesem Dauerversuch erhebliche Spuren von gebildetem Polymer.

### Vergleichsbeispiel 2

In einer Anlage, bestehend aus einem Umlaufverdampfer und einer Kolonne wurde Hydroxypropylmethacrylat in einem Dauerversuch, der über 15 Tage durchgeführt wurde, aufgereinigt. Die Anlage wurde mit einem Druck von circa 2mbar - 3 mbar und einer Temperatur von circa 67°C - 79°C betrieben. Die Zulaufmenge, mit der das aus einer zuvor durchgeführten Produktion erhaltene Hydroxypropylmethacrylat in die Mitte der Kolonne eingeleitet wurde, betrug im Mittel 1,486 kg/h. Der Massenstrom, mit dem die erhaltenen Brüden über Kopf aus der Kolonne ausgeleitet wurden, betrug im Mittel 1,390 kg/h. Der erhaltene Rückstand von im Mittel 0,096 kg/h wurde aus dem Umlaufverdampfer aus der Anlage ausgeschleust.

Das in die Anlage eingeleitete Hydroxypropylmethacrylat wies eine Reinheit von circa 95,0 %. Das aufgereinigte Hydroxypropylmethacrylat zeigte eine Reinheit von 98,7 %. Die Ausbeute betrug 93,5 %.

Die Anlage zeigte nach diesem Dauerversuch erhebliche Spuren von gebildetem Polymer.

### Vergleichsbeispiel 3

In einer Anlage, bestehend aus einem Umlaufverdampfer und einer Kolonne wurde 2-Hydroxyethylacrylat in einem Dauerversuch, der über 8 Tage durchgeführt wurde, aufgereinigt. Die Anlage wurde mit einem Druck von circa 2mbar - 3 mbar und einer Temperatur von circa 56°C - 70°C betrieben. Die Zulaufmenge, mit der das aus einer zuvor durchgeführten Produktion erhaltene 2-Hydroxyethylacrylat in die Mitte der Kolonne eingeleitet wurde, betrug im Mittel 1,330 kg/h. Der Massenstrom, mit dem die erhaltenen Brüden über Kopf aus der Kolonne ausgeleitet wurden, betrug im Mittel 0,950 kg/h. Der erhaltene Rückstand von im Mittel 0,380 kg/h wurde aus dem Umlaufverdampfer aus der Anlage ausgeschleust.

Das in die Anlage eingeleitete 2-Hydroxyethylacrylat wies eine Reinheit von circa 85,3 %. Das aufgereinigte 2-Hydroxyethylacrylat zeigte eine Reinheit von 98,5 %. Die Ausbeute betrug 71,4 %.
Die Anlage musste nach 8 Tagen aufgrund erheblicher Mengen von gebildetem Polymer abgestellt werden.

## Patentansprüche

1. Verfahren zur Aufreinigung von ungesättigten Verbindungen, wobei die Aufreinigung in einer Anlage durchgeführt wird, die mindestens zwei Verdampfer aufweist und die Verdampfer so verbunden sind, dass ein Teil der ungesättigten Verbindung in einem Kreislauf geführt wird, wobei die im ersten Verdampfer kondensierten Brüden isoliert werden und die im zweiten Verdampfer kondensierten Brüden in den ersten Verdampfer eingeleitet werden, welches **dadurch gekennzeichnet ist, dass** der Massenstrom, mit dem die kondensierten Brüden in dem ersten Verdampfer aus der zu reinigenden Mischung isoliert werden, kleiner ist als der Massenstrom, mit dem die kondensierten Brüden aus dem zweiten Verdampfer in den ersten Verdampfer eingeleitet werden.

2. Verfahren gemäß Anspruch 1, **dadurch gekennzeichnet, dass** die ungesättigte Verbindung ein Glykolester ist.

3. Verfahren gemäß Anspruch 2, **dadurch gekennzeichnet, dass** der Glykolester ein Hydroxyalkyl(meth)acrylat ist.

4. Verfahren gemäß mindestens einem der vorhergehenden Ansprüche, **dadurch gekennzeichnet, dass** das Verhältnis des Massenstromes, mit dem die im ersten Verdampfer kondensierten Brüden aus der zu reinigenden Mischung isoliert werden, zum Massenstrom, mit dem die kondensierten Brüden aus dem zweiten Verdampfer in den ersten Verdampfer eingeleitet werden, im Bereich von 0,4 bis 1,5 liegt.

5. Verfahren gemäß Anspruch 4, **dadurch gekennzeichnet, dass** das Verhältnis des Massenstromes, mit dem die im ersten Verdampfer kondensierten Brüden aus der zu reinigenden Mischung isoliert werden, zum Massenstrom, mit dem die kondensierten Brüden aus dem zweiten Verdampfer in den ersten Verdampfer eingeleitet werden, im Bereich von 0,5 bis 0,8 liegt.

6. Verfahren gemäß mindestens einem der vorhergehenden Ansprüche, **dadurch gekennzeichnet, dass** die Anlage mindestens drei Verdampfer aufweist, wobei ein Kreislauf zwischen dem ersten und dem zweiten Verdampfer und ein Kreislauf zwischen dem zweiten und dem dritten Verdampfer gebildet werden.

7. Verfahren gemäß Anspruch 6, **dadurch gekennzeichnet, dass** das Verhältnis des Massenstromes, mit dem die aus dem zweiten Verdampfer kondensierten Brüden in den ersten Verdampfer eingeleitet werden, zum Massenstrom, mit dem die kondensierten Brüden des dritten Verdampfers in den zweiten Verdampfer eingeleitet werden, im Bereich von 0,75 bis 35 liegt.

8. Verfahren gemäß mindestens einem der vorhergehenden Ansprüche, **dadurch gekennzeichnet, dass** mindestens ein Teil der zu reinigenden Mischung zunächst in den ersten Verdampfer eingeleitet wird.

9. Verfahren gemäß mindestens einem der vorhergehenden Ansprüche, **dadurch gekennzeichnet, dass** mindestens ein Teil der zu reinigenden Mischung zunächst in den zweiten Verdampfer eingeleitet wird.

10. Verfahren gemäß Anspruch 8 oder 9, **dadurch gekennzeichnet, dass** mindestens ein Teil der zu reinigenden Mischung zunächst in den ersten Verdampfer und ein weiterer Teil der zu reinigenden Mischung zunächst in den zweiten Verdampfer eingeleitet werden.

11. Verfahren gemäß Anspruch 10, **dadurch gekennzeichnet, dass** das Mengenverhältnis des in den ersten Verdampfer eingeleiteten Teils der zu reinigenden Mischung zu dem in den zweiten Verdampfer eingeleiteten Teil der zu reinigenden Mischung im Bereich von 9:1 bis 0,1:1 liegt.

12. Verfahren gemäß mindestens einem der vorhergehenden Ansprüche, **dadurch gekennzeichnet, dass** der erste Verdampfer ein Umlaufverdampfer ist.

13. Verfahren gemäß mindestens einem der vorhergehenden Ansprüche, **dadurch gekennzeichnet, dass** der zweite Verdampfer ein Umlaufverdampfer ist.

14. Verfahren gemäß mindestens einem der vorhergehenden Ansprüche, **dadurch gekennzeichnet, dass** der erste und / oder zweite Verdampfer ein Zwangsumlaufverdampfer ist.

15. Verfahren gemäß mindestens einem der vorhergehenden Ansprüche, **dadurch gekennzeichnet, dass** der erste und / oder zweite Verdampfer ein Naturumlaufverdampfer ist.

16. Verfahren gemäß mindestens einem der vorhergehenden Ansprüche, **dadurch gekennzeichnet, dass** der dritte Verdampfer ein Dünnschichtverdampfer ist.

17. Verfahren gemäß mindestens einem der vorhergehenden Ansprüche, **dadurch gekennzeichnet, dass** das Edukt bei einem Druck im Bereich von 0,1 mbar bis 20 mbar in die Gasphase überführt wird.

18. Verfahren gemäß mindestens einem der vorhergehenden Ansprüche, **dadurch gekennzeichnet, dass** die ungesättigte Verbindung bei einer Temperatur im Bereich von 40 °C bis 120 °C in die Gasphase überführt wird.

19. Verfahren gemäß Anspruch 18, **dadurch gekennzeichnet, dass** der Gasbelastungsfaktor im Bereich von 0,8 bis 3,0 Pa^{0,5} liegt.

20. Anlage zur Durchführung des Verfahrens gemäß mindestens einem der Ansprüche 1 bis 19, **dadurch gekennzeichnet, dass** die Anlage mindestens drei Verdampfer aufweist, die so miteinander verbunden sind, dass der in der flüssigen Phase verbliebene Rückstand des ersten Verdampfers in den zweiten Verdampfer geleitet werden kann, die kondensierten Brüden des zweiten Verdampfers in den ersten Verdampfer und der in der flüssigen Phase verbliebene Rückstand des zweiten Verdampfers in den dritten Verdampfer geleitet werden kann, und die kondensierten Brüden des dritten Verdampfers in den zweiten Verdampfer geleitet werden können.

21. Anlage gemäß Anspruch 20, **dadurch gekennzeichnet, dass** die Anlage mindestens zwei Umlaufverdampfer und einen Dünnschichtverdampfer aufweist.

22. Anlage gemäß Anspruch 20 oder 21, **dadurch gekennzeichnet, dass** die Anlage mindestens drei Kolonnen aufweist.

23. Anlage gemäß Anspruch 21, **dadurch gekennzeichnet, dass** die Kolonne bei einem F-Faktor im Bereich von 0,8 bis 3,0 Pa^{0,5} vorzugsweise im Bereich von 1,5 bis 2,0 Pa^{0,5} einen Druckabfall im Bereich von 0,5 bis 10 Pa^{0,5} vorzugsweise im Bereich von 1 bis 5 mbar aufweist.

24. Anlage gemäß Anspruch 21 oder 22, **dadurch gekennzeichnet, dass** die Kolonne einen Tröpfchenabscheider (Demister) aufweist.

## Claims

1. Process for purifying unsaturated compounds, said purification being performed in a plant which comprises at least two evaporators which are connected in such a way that a portion of the unsaturated compound is circulated, the vapours condensed in the first evaporator being isolated and the vapours condensed in the second evaporator being introduced into the first evaporator, which is **characterized in that** the mass flow with which the condensed vapours are isolated from the mixture to be purified in the first evaporator is less than the mass flow with which the condensed vapours from the second evaporator are introduced into the first evaporator.

2. Process according to Claim 1, **characterized in that** the unsaturated compound is a glycol ester.

3. Process according to Claim 2, **characterized in that** the glycol ester is a hydroxyalkyl (meth)acrylate.

4. Process according to at least one of the preceding claims, **characterized in that** the ratio of the mass flow with which the vapours condensed in the first evaporator are isolated from the mixture to be purified to the mass flow with which the condensed vapours from the second evaporator are introduced into the first evaporator is in the range from 0.4 to 1.5.

5. Process according to Claim 4, **characterized in that** the ratio of the mass flow with which the vapours condensed in the first evaporator are isolated from the mixture to be purified to the mass flow with which the condensed vapours from the second evaporator are introduced into the first evaporator is in the range from 0.5 to 0.8.

6. Process according to at least one of the preceding claims, **characterized in that** the plant comprises at least three evaporators, in which case one circuit is formed between the first and the second evaporator and one circuit between the second and the third evaporator.

7. Process according to Claim 6, **characterized in that** the ratio of the mass flow with which the condensed vapours from the second evaporator are introduced into the first evaporator to the mass flow with which the condensed vapours of the third evaporator are introduced into the second evaporator is in the range from 0.75 to 35.

8. Process according to at least one of the preceding claims, **characterized in that** at least a portion of the mixture to be purified is first introduced into the first evaporator.

9. Process according to at least one of the preceding claims, **characterized in that** at least a portion of the mixture to be purified is first introduced into the second evaporator.

10. Process according to Claim 8 or 9, **characterized in that** at least a portion of the mixture to be purified is first introduced into the first evaporator and a further portion of the mixture to be purified is first introduced into the second evaporator.

11. Process according to Claim 10, **characterized in that** the quantitative ratio of the portion of the mixture to be purified introduced into the first evaporator to the portion of the mixture to be purified introduced into the second evaporator is in the range from 9:1 to 0.1:1.

12. Process according to at least one of the preceding claims, **characterized in that** the first evaporator is a circulation evaporator.

13. Process according to at least one of the preceding claims, **characterized in that** the second evaporator is a circulation evaporator.

14. Process according to at least one of the preceding claims, **characterized in that** the first and/or second evaporator is a forced-circulation evaporator.

15. Process according to at least one of the preceding claims, **characterized in that** the first and/or second evaporator is a natural-circulation evaporator.

16. Process according to at least one of the preceding claims, **characterized in that** the third evaporator is a thin-film evaporator.

17. Process according to at least one of the preceding claims, **characterized in that** the reactant is converted to the gas phase at a pressure in the range from 0.1 mbar to 20 mbar.

18. Process according to at least one of the preceding claims, **characterized in that** the unsaturated compound is converted to the gas phase at a temperature in the range from 40°C to 120°C.

19. Process according to Claim 18, **characterized in that** the gas loading factor is in the range from 0.8 to 3.0 Pa^{0,5}_{.}

20. Plant for performing the process according to at least one of Claims 1 to 19, **characterized in that** the plant comprises at least three evaporators which are connected to one another such that the residue of the first evaporator remaining in the liquid phase can be passed into the second evaporator, the condensed vapours of the second evaporator can be passed into the first evaporator and the residue of the second evaporator remaining in the liquid phase can be passed into the third evaporator, and the condensed vapours of the third evaporator can be passed into the second evaporator.

21. Plant according to Claim 20, **characterized in that** the plant comprises at least two circulation evaporators and one thin-film evaporator.

22. Plant according to Claim 20 or 21, **characterized in that** the plant has at least three columns.

23. Plant according to Claim 21, **characterized in that** the column, at an F factor in the range from 0.8 to 3.0 Pa^{0.5}, preferably in the range from 1.5 to 2.0 Pa^{0.5}, has a pressure drop in the range from 0.5 to 10 mbar, preferably in the range from 1 to 5 mbar.

24. Plant according to Claim 21 or 22, **characterized in that** the column comprises a droplet separator (demister).

## Revendications

1. Procédé de purification de composés insaturés, la purification étant réalisée dans une installation qui présente au moins deux évaporateurs et les évaporateurs étant raccordés de manière telle qu'une partie du composé insaturé est guidée dans un circuit, les vapeurs condensées dans le premier évaporateur étant isolées et les vapeurs condensées dans le deuxième évaporateur étant introduites dans le premier évaporateur, **caractérisé en ce que** le flux massique par lequel les vapeurs condensées dans le premier évaporateur sont isolées du mélange à purifier est inférieur au flux massique par lequel les vapeurs condensées du deuxième évaporateur sont introduites dans le premier évaporateur.

2. Procédé selon la revendication 1, **caractérisé en ce que** le composé insaturé est un ester de glycol.

3. Procédé selon la revendication 2, **caractérisé en ce que** l'ester de glycol est un (méth)acrylate d'hydroxyalkyle.

4. Procédé selon au moins l'une quelconque des revendications précédentes, **caractérisé en ce que** le rapport du flux massique, par lequel les vapeurs condensées dans le premier évaporateur sont isolées du mélange à purifier au flux massique par lequel les vapeurs condensées du deuxième évaporateur sont introduites dans le premier évaporateur se situe dans la plage de 0,4 à 1,5.

5. Procédé selon la revendication 4, **caractérisé en ce que** le rapport du flux massique, par lequel les vapeurs condensées dans le premier évaporateur sont isolées du mélange à purifier au flux massique par lequel les vapeurs condensées du deuxième évaporateur sont introduites dans le premier évaporateur se situe dans la plage de 0,5 à 0,8.

6. Procédé selon au moins l'une quelconque des revendications précédentes, **caractérisé en ce que** l'installation présente au moins trois évaporateurs, un circuit étant formé entre le premier et le deuxième évaporateur et un circuit étant formé entre le deuxième et le troisième évaporateur.

7. Procédé selon la revendication 6, **caractérisé en ce que** le rapport du flux massique par lequel les vapeurs condensées du deuxième évaporateur sont introduites dans le premier évaporateur au flux massique par lequel les vapeurs condensées du troisième évaporateur sont introduites dans le deuxième évaporateur se situe dans la plage de 0,75 à 35.

8. Procédé selon au moins l'une quelconque des revendications précédentes, **caractérisé en ce qu'**au moins une partie du mélange à purifier est d'abord introduite dans le premier évaporateur.

9. Procédé selon au moins l'une quelconque des revendications précédentes, **caractérisé en ce qu'**au moins une partie du mélange à purifier est d'abord introduite dans le deuxième évaporateur.

10. Procédé selon la revendication 8 ou 9, **caractérisé en ce qu'**au moins une partie du mélange à purifier est d'abord introduite dans le premier évaporateur et une autre partie du mélange à purifier est d'abord introduite dans le deuxième évaporateur.

11. Procédé selon la revendication 10, **caractérisé en ce que** le rapport de quantités de la partie du mélange à purifier introduite dans le premier évaporateur à la partie du mélange à purifier introduite dans le deuxième évaporateur se situe dans la plage de 9:1 à 0,1:1.

12. Procédé selon au moins l'une quelconque des revendications précédentes, **caractérisé en ce que** le premier évaporateur est un évaporateur à circulation.

13. Procédé selon au moins l'une quelconque des revendications précédentes, **caractérisé en ce que** le deuxième évaporateur est un évaporateur à circulation.

14. Procédé selon au moins l'une quelconque des revendications précédentes, **caractérisé en ce que** le premier et/ou le deuxième évaporateur est un évaporateur à circulation forcée.

15. Procédé selon au moins l'une quelconque des revendications précédentes, **caractérisé en ce que** le premier et/ou le deuxième évaporateur est un évaporateur à circulation naturelle.

16. Procédé selon au moins l'une quelconque des revendications précédentes, **caractérisé en ce que** le troisième évaporateur est un évaporateur à couche mince.

17. Procédé selon au moins l'une quelconque des revendications précédentes, **caractérisé en ce que** le produit de départ est transféré dans la phase gazeuse à une pression dans la plage de 0,1 mbar à 20 mbars.

18. Procédé selon au moins l'une quelconque des revendications précédentes, **caractérisé en ce que** le composé insaturé est transféré dans la phase gazeuse à une température dans la plage de 40°C à 120°C.

19. Procédé selon la revendication 18, **caractérisé en ce que** le facteur de charge du gaz se situe dans la plage de 0,8 à 3,0 Pa^{0,5}.

20. Installation pour la réalisation du procédé selon au moins l'une quelconque des revendications 1 à 19, **caractérisée en ce que** l'installation présente au moins trois évaporateurs, qui sont raccordés l'un à l'autre de manière telle que le résidu resté dans la phase liquide du premier évaporateur peut être guidé dans le deuxième évaporateur, les vapeurs condensées du deuxième évaporateur peuvent être guidées dans le premier évaporateur et le résidu resté dans la phase liquide du deuxième évaporateur peut être guidé dans le troisième évaporateur et les vapeurs condensées du troisième évaporateur peuvent être guidées dans le deuxième évaporateur.

21. Installation selon la revendication 20, **caractérisée en ce que** l'installation présente au moins deux évaporateurs à circulation et un évaporateur à couche mince.

22. Installation selon la revendication 20 ou 21, **caractérisée en ce que** l'installation présente au moins trois colonnes.

23. Installation selon la revendication 21, **caractérisée en ce que** la colonne présente, à un facteur F dans la plage de 0,8 à 3,0 Pa^{0,5}, de préférence dans la plage de 1,5 à 2,0 Pa^{0,5}, présente une perte de charge dans la plage de 0,5 à 10 mbars de préférence dans la plage de 1 à 5 mbars.

24. Installation selon la revendication 21 ou 22, caractérisée e n c e que la colonne présente un séparateur de gouttes (Demister).
